(19) Europäisches Patentamt
European Patent Office
Office européen des brevets

(11) Numéro de publication : **0 044 575**
**B1**

(12) **FASCICULE DE BREVET EUROPÉEN**

(45) Date de publication du fascicule du brevet : **21.03.84**

(51) Int. Cl.³ : **C 07 J 7/00**, C 07 D317/72

(21) Numéro de dépôt : **81200353.1**

(22) Date de dépôt : **14.11.79**

(60) Numéro de publication de la demande initiale en application de l'article 76 CBE : **0012641**

(54) **Produits intermédiaires dans la synthèse de 19-nor stéroïdes substitués en position 2.**

(30) Priorité : **13.12.78 FR 7835046**

(43) Date de publication de la demande : **27.01.82 Bulletin 82/04**

(45) Mention de la délivrance du brevet : **21.03.84 Bulletin 84/12**

(84) Etats contractants désignés : **AT BE CH DE FR GB IT NL SE**

(56) Documents cités :
**CH-A- 473 106**
**FR-A- 1 476 572**
**Le dossier contient des informations techniques présentées postérieurement au dépôt de la demande et ne figurant pas dans le présent fascicule.**

(73) Titulaire : **ROUSSEL-UCLAF**
**35, boulevard des Invalides**
**F-75007 Paris (FR)**

(72) Inventeur : **Nedelec, Lucien**
**45, boulevard de l'Ouest**
**F-93340 Le Raincy (FR)**
Inventeur : **Torelli, Vesperto**
**5, rue de la Convention**
**F-94700 Maisons-Alfort (FR)**
Inventeur : **Fournex, Robert**
**8, rue du Commandant Rivière**
**F-75008 Paris (FR)**

(74) Mandataire : **Tonnellier, Marie-José et al**
**102, route de Noisy Boîte postale no 9**
**F-93230 Romainville (FR)**

**EP 0 044 575 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

# Produits intermédiaires dans la synthèse de 19-nor stéroïdes substitués en position 2

La présente invention concerne de nouveaux intermédiaires de structure 19-nor stéroïde et substitués en position 2 ainsi que leur procédé de préparation.

On connaissait d'après les brevets français 1.476.572 et suisse 473.106 des produits 19-nor comportant un radical acétyle ou un radical 1-hydroxy éthyle en position 17β. Cependant les produits de l'art antérieur ne comportent aucune substitution en position 2.

L'invention a pour objet les produits de formule Ii :

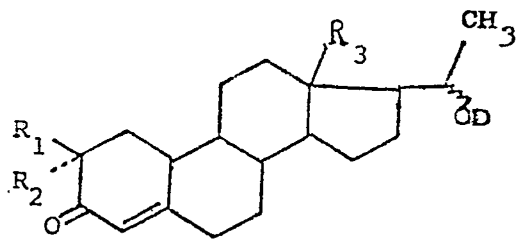

(Ii)

sous forme d'isomères 20R ou 20S ou de leur mélange, formule dans laquelle $R_1$ et $R_2$ identiques ou différents représentent un atome d'hydrogène, un radical alkyle renfermant de 1 à 4 atomes de carbone ou un radical alkényle ou alkynyle renfermant de 2 à 6 atomes de carbone, étant entendu que seul un des substituant $R_1$ et $R_2$ peut représenter un atome d'hydrogène, ou bien $R_1$ et $R_2$ forment ensemble un radical cycloalkyle renfermant de 3 à 8 atomes de carbone ; $R_3$ représente un radical alkyle renfermant de 2 à 4 atomes de carbone ; OD représentant un groupement éther.

Lorsque $R_1$ ou $R_2$ représente un radical alkyle, il s'agit de préférence du radical méthyle ou éthyle.

Lorsque $R_1$ ou $R_2$ représente un radical alkényle, il s'agit de préférence du radical allyle.

Lorsque $R_1$ ou $R_2$ représente un radical alkynyle, il s'agit de préférence du radical 2-propynyle.

Lorsque $R_1$ ou $R_2$ forment ensemble un radical cycloalkyle, il s'agit de préférence du radical cyclopropyle, cyclobutyle, cyclopentyle ou cyclohexyle.

L'invention a particulièrement pour objet les produits de formule Ii tels que définis précédemment, pour lesquels $R_3$ représente un radical éthyle ainsi que ceux pour lesquels $R_1$ et $R_2$ représentent chacun un radical méthyle.

L'invention a plus spécialement pour objet le (2'R,S,20R,S) 2,2-diméthyl 13-éthyl 20-/2'-(tétrahydro-pyrannyl) oxy/18,19-dinor pregn-4-èn 3-one.

L'invention a également pour objet un procédé de préparation des produits de formule Ii, caractérisé en ce que l'on condense, par réaction de WITTIG, un composé de formule II :

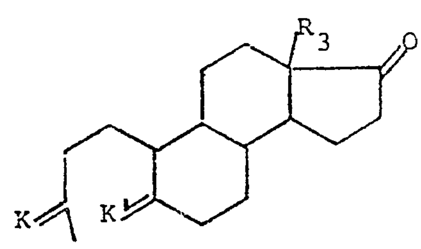

(II)

dans laquelle K et K' représentent un groupement cétone bloquée sous forme de cétal et $R_3$ représente un radical alkyle renfermant de 2 à 4 atomes de carbone, avec un halogénure de triphényl éthyl phosphonium de formule III :

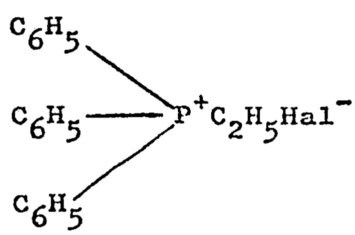

(III)

dans laquelle Hal représente un atome d'halogène, en présence d'une base forte, pour obtenir un composé de formule IV :

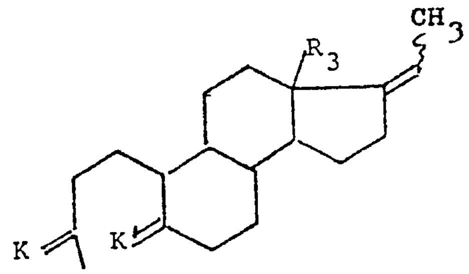

(IV)

sous forme d'un mélange d'isomères cis et trans, que l'on sépare, si désiré, en chacun des isomères, puis soumet soit chacun des isomères de formule IV, soit leur mélange, à l'action d'un agent d'hydroboration, puis à celle d'un agent d'oxydation en milieu alcalin et enfin à celle d'un agent de décétalisation et à celle d'un agent de cyclisation, pour obtenir un composé de formule V :

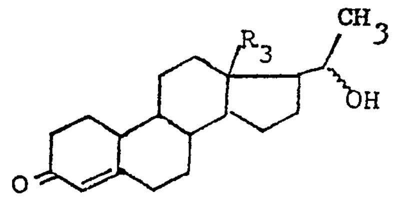

(V)

sous forme d'isomères 20R ou 20S ou de leur mélange, soumet soit chacun des isomères de formule V, soit leur mélange, à l'action d'un agent de blocage de la fonction alcool sous forme d'éther facilement clivable, pour obtenir un composé de formule VI :

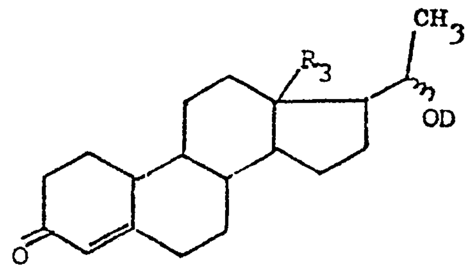

(VI)

sous forme d'isomères 20R ou 20S ou sous forme de leur mélange, dans laquelle OD représente un groupement éther, soumet soit chacun des isomères de formule VI, soit leur mélange, à l'action d'un halogénure d'alkyle, d'alkényle ou d'alkynyle en présence d'un agent basique à basse température, pour obtenir un composé de formule Ii :

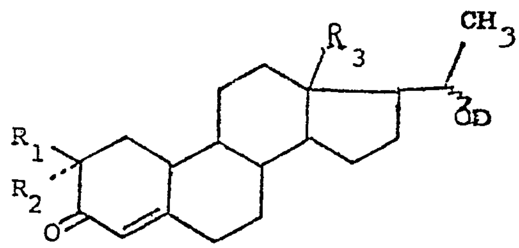

(Ii)

dans laquelle $R_1$ et $R_2$ conservent la même signification que précédemment, sous forme d'isomère 20R ou 20S ou sous forme de leur mélange.

Dans un mode de réalisation préférée du procédé de l'invention :

— $R_3$ représente un radical éthyle.

— K et K' représentent un groupement alkylcétal cyclique ayant de 2 à 4 atomes de carbone et, notamment, l'éthylène cétal ou le propylène cétal ou encore un dialkylcétal par exemple le diméthyl ou le diéthylcétal.

La réaction de WITTIG est réalisée dans les conditions opératoires décrites dans « Organic Reactions » *14*, 270, (1965).

— L'halogénure d'éthyltriphénylphosphonium utilisé est le bromure d'éthyltriphénylphosphonium.

— La base forte utilisée au cours de la réaction de WITTIG est un alcoolate de métal alcalin, par exemple, le téramylate de sodium ou de potassium ou le terbutylate de sodium ou de potassium.

— La réaction de WITTIG est effectuée au sein d'un solvant tel que le benzène, le toluène, le tétrahydrofuranne, le dioxanne ou le diméthyl sulfoxyde.

— La réaction d'hydratation des oléfines de formule IV est réalisée suivant la « méthode de BROWN » décrite par exemple dans « Organic Reactions » volume *13*, 1, 1963).

— L'agent d'hydroboration est le diborane, ou le diborane formé « *in situ* » par réaction du borohydrure de sodium sur le trifluorure de bore, ou encore un dialkylborane comme le 9-bora-bicyclo/3, 3-1/nonane ou le diisoamylborane.

— L'agent d'oxydation en milieu alcalin utilisé est l'eau oxygénée au sein d'une solution de soude ou de potasse.

— On utilise de préférence un seul réactif comme agent de décétalisation et de cyclisation, à savoir un acide fort tel que l'acide chlorhydrique ou l'acide sulfurique, mais on peut utiliser également deux agents différents. On peut par exemple utiliser un acide faible comme l'acide acétique et ensuite un agent basique comme la soude ou la potasse.

— Les isomères obtenus lors de la mise en œuvre du procédé peuvent être séparés par les méthodes classiques de cristallisation ou de chromatographie.

— L'agent de blocage de la fonction alcool est le dihydropyranne.

— $R_1$ et $R_2$ représentent chacun un radical méthyle et l'on introduit $R_1$ et $R_2$ par gem-diméthylation effectuée au moyen de l'iodure de méthyle, l'agent basique utilisé étant un alcoolate alcalin comme le terbutylate de potassium.

La réaction de gem-diméthylation est avantageusement mise en œuvre dans un solvant aprotique comme par exemple le tétrahydrofuranne.

Les produits de la présente demande constituent des intermédiaires pour la préparation des produits de formule I' :

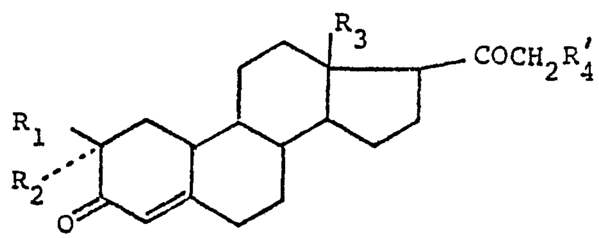

(I')

dans laquelle $R_3$ représente un radical alkyle renfermant de 2 à 4 atomes de carbone, $R'_4$ représente un atome d'hydrogène, un radical hydroxyle, un radical acyloxy renfermant de 1 à 18 atomes de carbone ou un radical :

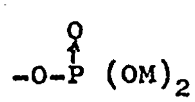

dans lequel M représente un atome d'hydrogène, un atome de sodium ou de potassium, et, ou bien $R_1$ et $R_2$, identiques ou différents, représentent un atome d'hydrogène, un radical alkyle renfermant de 1 à 4 atomes de carbone, ou un radical alkényle ou alkynyle renfermant de 2 à 6 atomes de carbone, étant entendu que seul un des substituants $R_1$ et $R_2$ peut représenter un atome d'hydrogène, ou bien $R_1$ et $R_2$ forment ensemble un radical cycloalkyle renfermant de 3 à 8 atomes de carbone.

Les composés de formule I' présentent d'intéressantes propriétés pharmacologiques ; ils sont en particulier des antagonistes de l'aldostérone et augmentent la diurèse hydrosodée avec conservation du potassium organique, tout en étant dénués d'effets secondaires. Il a été constaté en particulier qu'aux doses où les produits présentent une activité anti aldostérone intéressante, ceux-ci ne manifestent pas d'activité anti androgène ni d'activité anti estrogène. Ils peuvent donc être utilisés pour lutter contre l'hypertension artérielle et les insuffisances cardiaques.

Le procédé de préparation des produits de formule I' à partir des produits de formule II de la présente demande est le suivant :

On soumet soit chacun des isomères des produits de formule II soit leur mélange, à l'action d'un agent d'hydrolyse puis à celle d'un agent d'oxydation, pour obtenir un composé de formule $I_A$ :

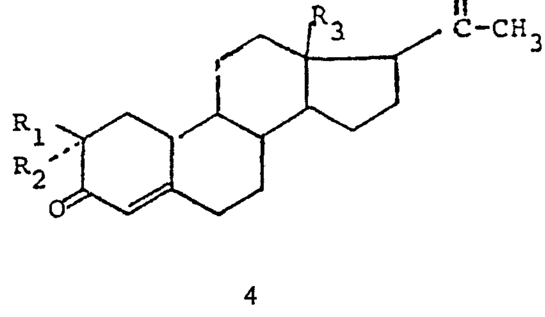

($I_A$)

que l'on soumet, si désiré, ou bien à l'action du tétracétate de plomb, ou bien à l'action d'un agent d'oxalylation puis à celle d'un agent d'halogénation, pour obtenir un dérivé 21-halogéné correspondant que l'on traite par un agent d'acétoxylation, pour obtenir un composé de formule $I_B$ :

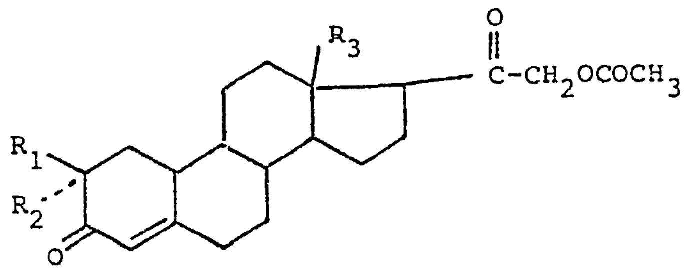

($I_B$)

que l'on soumet, si désiré, à l'action d'un agent de saponification, pour obtenir un composé de formule $I_C$ :

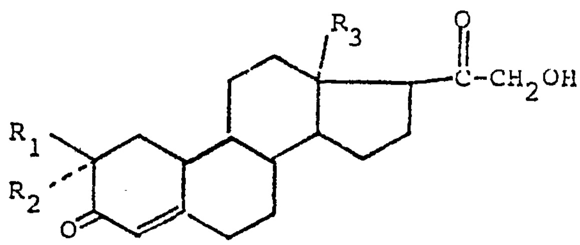

($I_C$)

que l'on soumet, si désiré, soit à l'action d'un acide ou d'un dérivé fonctionnel d'acide renfermant de 1 à 18 atomes de carbone, pour obtenir un composé de formule $I_D$ :

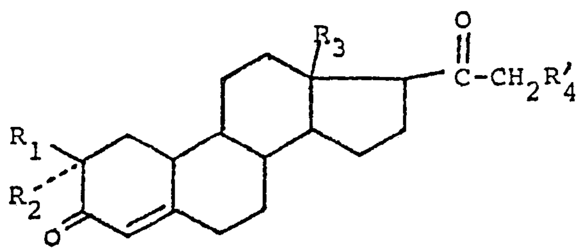

($I_D$)

dans laquelle $R'_4$ représente un radical acyloxy renfermant de 1 à 18 atomes de carbone, soit à l'action d'un dérivé fonctionnel de l'acide phosphorique, pour obtenir un composé de formule $I_D$ dans laquelle $R'_4$ représente un radical :

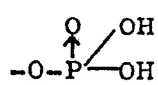

que, si désiré, l'on salifie.

— L'agent d'hydrolyse auquel on soumet le composé de la présente invention de formule Ii est de préférence un acide comme l'acide chlorhydrique, l'acide sulfurique, l'acide acétique ou l'acide paratoluène sulfonique.

— L'agent d'oxydation auquel on soumet le composé de la présente invention de formule Ii est le réactif d'HEILBRON-JONES, c'est-à-dire, l'anhydride chromique en solution dans l'acide sulfurique dilué.

— La réaction avec le tétracétate de plomb a lieu en présence d'éthérate de trifluorure de bore.

— L'agent d'oxalylation est l'oxalate d'éthyle.

— L'agent d'halogénation est l'iode ou le brome.

— L'agent d'acétoxylation est l'acétate de potassium.

— L'agent de saponification est de préférence une base alcaline comme la soude, la potasse, le carbonate de potassium ou carbonate acide de potassium et la réaction de saponification est réalisée de préférence au sein d'un alcool inférieur comme le méthanol ou l'éthanol.

— Le dérivé fonctionnel d'acide utilisé est de préférence un halogénure d'acide par exemple le bromure ou le chlorure ou bien un anhydride d'acide.

— La préparation du dérivé phosphoré est effectuée selon des méthodes classiques décrites dans la littérature.

La salification du dérivé phosphoré est effectuée par l'hydroxyde de sodium ou de potassium.

Les produits de formule II, utilisés comme produits de départ du procédé de l'invention sont des produits connus d'une façon générale et qui peuvent être préparés selon les procédés décrits dans le brevet français 1 490 590 et dans le brevet danois 136 115.

Les exemples suivants illustrent l'invention sans toutefois la limiter.

Exemple 1

2,2-diméthyl 13-éthyl 18,19-dinor pregn 4-èn 3,20-dione

Stade A : bis (1,2-éthanediyl)acétal cyclique de 13-éthyl 18,19-dinor 4,5-seco pregn 17(20)-èn 3,5-dione (mélange d'isomères Z et E)

On introduit 51,2 g de bromure de triphényl éthyl phosphonium dans 136 $cm^3$ de diméthylsulfoxyde renfermant 16,8 g de terbutylate de potassium. On ajoute ensuite 27,2 g de bis (1,2-éthanediylacétal)cyclique de 13-éthyl 18,19-dinor 4,5 seco androstane 17-one. On chauffe sous agitation et sous courant d'azote à 50 °C, pendant 65 heures et verse le mélange réactionnel dans 1,5 l d'eau. On extrait à l'éther. La phase organique est lavée à l'eau, séchée et concentrée sous pression réduite à petit volume. On sépare par filtration l'oxyde de triphénylphosphonium puis concentre le filtrat à sec.

Le résidu est purifié par chromatographie sur silice en éluant avec un mélange de cyclohexane-acétate d'éthyle (8-2). On obtient ainsi 27,35 g du produit recherché que l'on utilise tel quel dans le stade suivant.

Stade B : 13-éthyl 20(R,S)-hydroxy 18,19-dinor pregn 4-èn 3-one

On introduit sous atmosphère d'argon, 1,9 g d'hydroborure de sodium dans 19 $cm^3$ de tétrahydrofuranne anhydre. On refroidit la suspension à − 10 °C − 15 °C et ajoute une solution renfermant 6,25 $cm^3$ d'éthérate de trifluorure de bore dans 19 $cm^3$ de tétrahydrofuranne anhydre. On agite la suspension pendant une heure à − 5 °C puis ajoute, en 10 minutes, une solution de 10 g du produit obtenu au stade A en solution dans 30 $cm^3$ de tétrahydrofuranne anhydre. On amène le mélange réactionnel à température ambiante et agite pendant 1 heure 30. On refroidit ensuite le mélange réactionnel à − 10 °C et ajoute très lentement 30 $cm^3$ de lessive de soude puis 30 $cm^3$ d'eau. On décante la phase aqueuse.

On lave la phase organique deux fois avec de la soude 3N et lui ajoute 100 $cm^3$ de soude 3N. On émulsionne les deux phases par agitation et ajoute, lentement, à température ambiante 50 $cm^3$ de perhydrol à 33 %. On maintient l'agitation pendant 45 minutes puis décante la phase aqueuse et l'extrait avec de l'acétate d'éthyle. Les phases organiques jointes sont lavées avec une solution de thiosulfate de sodium à 10 %, séchées et concentrées à sec sous pression réduite. On ajoute au résidu 100 $cm^3$ d'éthanol et 50 $cm^3$ d'acide chlorhydrique environ 5N et chauffe la solution obtenue au reflux pendant 50 minutes. On dilue ensuite à l'eau et extrait au chloroforme. On lave à l'eau la solution chloroformique, la sèche et évapore sous pression réduite. On obtient 9,8 g de produit que l'on purifie par cristallisation dans l'éther isopropylique. On obtient ainsi 5,1 g de produit recherché fondant à 140 °C, qui après recristallisation dans le méthanol aqueux, fond à 142 °C.

Par chromatographie de ce produit sur silice dans le mélange cyclohexane-acétate d'éthyle (1-1), on obtient l'isomère 20 R pur fondant à 150 °C et l'isomère 20 S pur fondant à 169 °C.

Stade C : 13-éthyl (2'RS,20RS) 20-/2'-(tétrahydropyrannyl) oxy/18,19-dinor pregn 4-èn 3-one

On dissout 13,42 g de 13-éthyl 20(R,S)hydroxy 18,19-dinor pregn 4-èn 3-one dans une solution renfermant 135 $cm^3$ de benzène anhydre et 27 $cm^3$ de dihydropyranne. On ajoute 135 mg d'acide paratoluènesulfonique et abandonne la solution une heure à la température ambiante. On ajoute 1 $cm^3$ de triéthylamine, lave la phase organique à l'eau. On sèche et évapore sous pression réduite.

On obtient 19,1 g de résidu cristallisé renfermant le produit recherché que l'on utilise tel quel dans le stade suivant.

Stade D : (2'RS,20RS) 2,2-diméthyl 13-éthyl 20-/2'-(tétrahydropyrannyl)oxy/18,19-dinor pregn 4-èn 3-one

On dissout 6,6 g du produit obtenu au stade C dans une solution renfermant 50 $cm^3$ de tétrahydrofuranne anhydre et 11 $cm^3$ d'iodure de méthyle. On refroidit la solution à − 65 °C sous atmosphère d'azote et ajoute en 20 minutes, 9,2 g de terbutylate de potassium en solution dans 45 $cm^3$ de tétrahydrofuranne anhydre. On maintient la suspension sous agitation pendant encore 30 minutes à 65 °C, puis la dilue avec de l'eau. On extrait avec du chloroforme, lave la phase organique à l'eau, la sèche et la distille à sec. On obtient 7,1 g d'extrait sec renfermant le produit recherché que l'on utilise tel quel dans le stade suivant.

Stade E : 2,2-diméthyl 13-éthyl 18,19-dinor pregn 4-èn 3,20-dione

On dissout les 7,1 g du produit obtenu au stade D dans une solution renfermant 70 $cm^3$ d'éthanol et 14 $cm^3$ d'acide chlorhydrique 2N. On chauffe à reflux pendant 1 heure puis dilue à l'eau et extrait au chlorure de méthylène.

L'extrait sec (6,9 g) est dissout dans 200 $cm^3$ d'acétone. On refroidit à 0°-5 °C, et ajoute, en 15 minutes, 5 $cm^3$ de réactif d'HEILBRON-JONES. On agite encore 15 minutes à 0°-5 °C puis détruit l'excès de réactif par addition de méthanol, dilue à l'eau et extrait au chlorure de méthylène. On obtient 5,9 g d'un produit que l'on purifie par chromatographie sur silice en éluant avec un mélange de cyclohexane-acétate d'éthyle (85-15). On obtient 2,6 g du produit recherché fondant à 179 °C après recristallisation dans un mélange chlorure de méthylène-éther isopropylique.

## Exemple 2

21-acétoxy 2,2-diméthyl 13-éthyl 18,19-dinor pregn 4-èn 3,20-dione

a) On dissout 1,920 g du produit obtenu à l'exemple 1 dans une solution renfermant 20 $cm^3$ de benzène anhydre et 2 $cm^3$ d'oxalate d'éthyle. On ajoute 450 mg d'hydrure de sodium en dispersion à 50 % dans l'huile minérale et une goutte d'éthanol pour faire démarrer la réaction. On agite la suspension pendant 50 minutes jusqu'à cessation du dégagement d'hydrogène. On acidifie ensuite le milieu réactionnel avec une solution d'acide chlorhydrique sec dans l'acétate d'éthyle. On filtre les sels minéraux et on évapore le filtrat sous pression réduite. On obtient le dérivé 21-oxalylé brut, partiellement cristallié, que l'on utilise tel quel ci-après.

b) On dissout le produit obtenu au paragraphe a/ dans une solution renfermant 24 $cm^3$ de diméthylformamide et 4,3 $cm^3$ de potasse méthanolique 1,3 N. On ajoute 2,15 g d'acétate de potassium. On refroidit la suspension obtenue à − 5 °C et ajoute, goutte à goutte, une solution renfermant 1,42 g d'iode dans un mélange de 3 $cm^3$ de tétrahydrofuranne et 15 $cm^3$ de diméthylformamide. On maintient sous agitation à − 5 °C pendant 35 minutes après la fin de l'addition. On obtient une suspension que l'on utilise telle quelle ci-après.

c) On ajoute 4,3 g d'acétate de potassium et chauffe la suspension sous agitation à 100 °C, pendant 30 minutes. On refroidit, dilue à l'eau et extrait à l'acétate d'éthyle. L'extrait est purifié par chromatographie sur silice (éluant : benzène-acétate d'éthyle 9-1). On obtient ainsi 1,170 g du produit recherché fondant à 112 °C après recristallisation dans l'éther isopropylique.

## Exemple 3

2,2-diméthyl 13-éthyl 21-hydroxy 18,19-dinor prègn 4-èn 3,20-dione

On dissout 3,4 g du produit obtenu à l'exemple 2, dans 68 $cm^3$ de méthanol. On chauffe la solution au reflux sous barbotage d'azote pendant 15 minutes, puis ajoute 840 mg de carbonate acide de potassium et 8,4 $cm^3$ d'eau. On maintient au reflux sous barbotage d'azote pendant 30 minutes, refroidit et ajoute 1 $cm^3$ d'acide acétique. On concentre sous pression réduite, dilue à l'eau et extrait au chlorure de méthylène. On lave la phase organique à l'eau et la sèche. On évapore à sec. On obtient 3,06 g de produit que l'on purifie par chromatographie sur silice (éluant : benzène-acétate d'éthyle 8-2). On obtient 3 g du produit recherché fondant à 138 °C après recristallisation dans l'éthanol aqueux.

## Exemple 4

2,2-diméthyl 13-éthyl 21-(4-pyridinyl carbonyloxy) 18,19-dinor prègn-4-ène 3,20-dione

On met en suspension sous gaz inerte, à − 10 °C, 541 mg d'acide isonicotinique dans 25 $cm^3$ de pyridine et 760 mg de chlorure de tosyle. On agite pendant 20 minutes à − 10 °C, ajoute 717 mg de 2,2-diméthyl 13-éthyl 21-hydroxy 18,19-dinor prègn-4-ène 3,20-dione et agite pendant une heure 30 à 10 °C. On verse dans l'eau, alcalinise par addition d'une solution aqueuse de bicarbonate de sodium, extrait au chlorure de méthylène, lave la phase organique avec une solution aqueuse de bicarbonate de sodium puis à l'eau, sèche et évapore à sec. On chromatographie le résidu sur silice en éluant au mélange benzène-acétate d'éthyle (6-4) et obtient 750 mg de produit attendu qui cristallise dans l'éther isopropylique. F = 156 °C.

## Exemple 5

Exemple de compositions pharmaceutiques

On a préparé des comprimés à 50 mg de produit de l'exemple 3, comme principe actif.

Produit de l'exemple 3 50 mg
Excipient (talc, amidon, stéarate de magnésium).

Etude pharmacologique

Etude de l'activité antialdostérone chez l'animal, du produit de l'exemple 3, nommé ci-après produit A.

L'étude a été effectuée au moyen d'un test inspiré de KAGAWA C.M. (Proc. Soc. Expt. Biol. Med. *99,* 705, (1958) et de MARCUS (Endocrinology *50,* 286, (1952)).

La technique utilisée est la suivante :

Des rats mâles Sprague Dawley SPF IFFA CREDO, de 180 g sont surrénalectomisés 7 jours avant la diurèse, sous anesthésie à l'Imalgène (Kétamine) par voie intrapéritonéale à raison de 100 mg/kg. Dès l'opération et jusqu'à la veille de l'expérience, ils reçoivent comme eau de boisson du sérum physiologique.

Les animaux sont mis à jeun dix-sept heures avant la diurèse, le sérum physiologique est alors remplacé par de l'eau glucosée à 5 %.

Le produit est administré par voie orale une heure avant la mise en cage.

Au moment de la mise en diurèse, les animaux reçoivent une surcharge hydrosaline par voie intrapéritonéale à raison de 5 ml par rat, de sérum physiologique à 9 % et d'autre part, 1 µg/kg de monoacétate d'aldostérone en solution alcoolique à 2,5 %, par voie sous-cutanée.

Les rats sont alors placés par deux en cage à diurèse, sans nourriture ni boisson, pendant quatre heures.

Au bout de ce temps, on effectue une miction forcée par pression sur la vessie et mesure le volume de l'urine recueillie.

Après rinçage soigneux des cages et de la verrerie, le volume des urines est amené à 50 $cm^3$. Sur cette solution on effectue le dosage du sodium et du potassium urinaires par photométrie de flamme à l'autoanalyseur.

Les résultats obtenus sont exprimés en pourcentage d'inhibition de l'activité de 1 µg/kg de monoacétate d'aldostérone injecté par voie sous-cutanée sur le log du rapport : concentration en sodium/concentration en potassium, selon la méthode de KAGAWA. Endocrinology 1960, *67* p. 125-132.

Il a été trouvé que le produit A à la dose de 2 mg/kg, par voie orale manifeste une très nette activité antialdostéronique ; le pourcentage d'inhibition est à cette dose de l'ordre de 60 %.

## Revendications

1. Les produits de formule Ii :

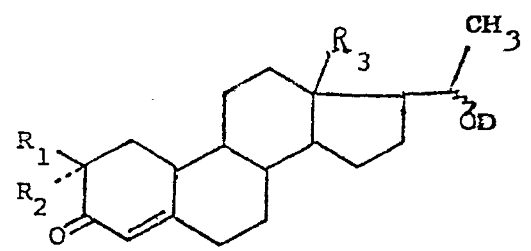

(Ii)

sous forme d'isomères 20R ou 20S ou de leur mélange, formule dans laquelle $R_1$ et $R_2$ identiques ou différents représentent un atome d'hydrogène, un radical alkyle renfermant de 1 à 4 atomes de carbone ou un radical alkényle ou alkynyle renfermant de 2 à 6 atomes de carbone, étant entendu que seul un des substituant $R_1$ et $R_2$ peut représenter un atome d'hydrogène, ou bien $R_1$ et $R_2$ forment ensemble un radical cycloalkyle renfermant de 3 à 8 atomes de carbone ; $R_3$ représente un radical alkyle renfermant de 2 à 4 atomes de carbone ; OD représentant un groupement éther.

2. La (2'R,S 20R,S) 2,2-diméthyl 13-éthyl 20-/2'-(tétrahydropyrannyl)oxy/18,19-dinor pregn-4-èn 3-one.

3. Procédé de préparation des produits tels que définis à la revendication 1, caractérisé en ce que l'on condense, par réaction de WITTIG, un composé de formule II :

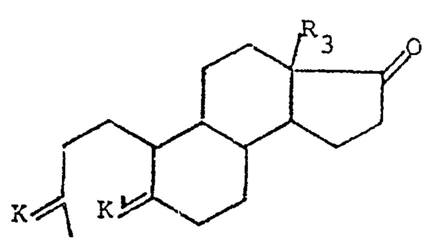

(II)

dans laquelle K et K' représentent un groupement cétone bloquée sous forme de cétal et $R_3$ représente un radical alkyle renfermant de 2 à 4 atomes de carbone, avec un halogénure de triphényléthylphosphonium de formule III :

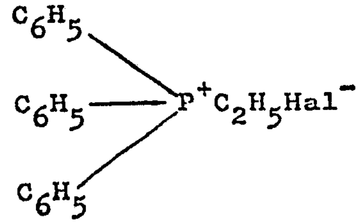

(III)

dans laquelle Hal représente un atome d'halogène, en présence d'une base forte, pour obtenir un composé de formule IV :

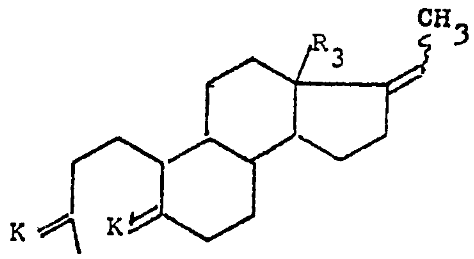

(IV)

sous forme d'un mélange d'isomères cis et trans que l'on sépare, si désiré, en chacun des isomères, puis soumet,

soit chacun des isomères de formule IV, soit leur mélange, à l'action d'un agent d'hydroboration, puis à celle d'un agent d'oxydation en milieu alcalin et enfin à celle d'un agent de décétalisation et à celle d'un agent de cyclisation, pour obtenir un composé de formule V :

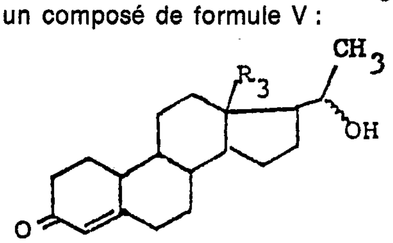

(V)

sous forme d'isomères 20R ou 20S ou de leur mélange, soumet,

soit chacun des isomères de formule V, soit leur mélange, à l'action d'un agent de blocage de la fonction alcool sous forme d'éther facilement clivable, pour obtenir un composé de formule VI :

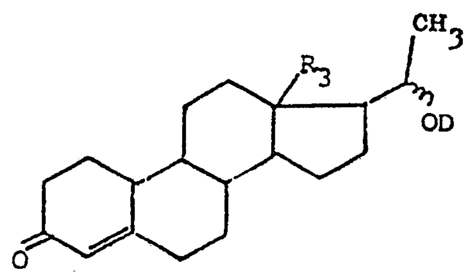

(VI)

sous forme d'isomères 20R ou 20S ou sous forme de leur mélange, dans laquelle OD représente un groupement éther, soumet,

soit chacun des isomères de formule VI, soit leur mélange, à l'action d'un halogénure d'alkyle, d'alkényle ou d'alkynyle, en présence d'un agent basique à basse température, pour obtenir un composé de formule VII :

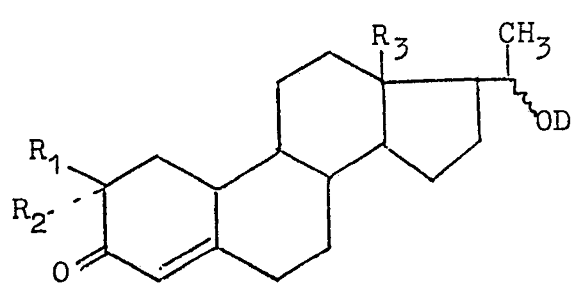

(VII)

dans laquelle $R_1$ et $R_2$ conservent la même signification que dans la revendication 1, sous forme d'isomère 20R ou 20S ou sous forme de leur mélange.

**Claims**

1. The products with the formula Ii :

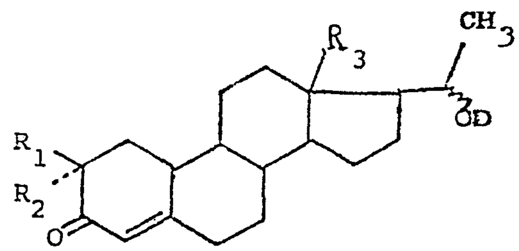

(Ii)

in the form of 20R or 20S isomers or their mixture, in which formula each of $R_1$ and $R_2$, identical or different, represents a hydrogen atom, an alkyl radical containing 1-4 carbon atoms or an alkenyl or alkynyl radical containing 2-6 carbon atoms, it being understood that only one of the substituents $R_1$ and $R_2$ can represent a hydrogen atom, or $R_1$ and $R_2$ together form a cyclo-alkyl radical containing 3-8 carbon atoms ; $R_3$ represents an alkyl radical containing 2-4 carbon atoms ; OD representing an ether group.

2. (2'R,S,20R,S)-2,2-dimethyl-13-ethyl-20-[2'-(tetrahydropyrannyl)oxy]18,19-dinor-pregn-4-en-3-one.

3. Preparation process for the products defined in Claim 1, characterized in that a compound with the formula II :

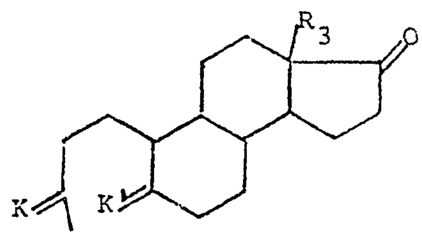

(II)

in which K and K' represent a blocked ketone group in ketal form and $R_3$ represents an alkyl radical containing 2-4 carbon atoms is condensed, by WITTIG reaction, with a triphenylethylphosphonium halide with the formula III :

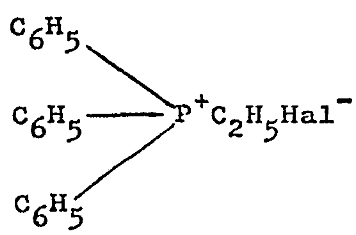

(III)

in which Hal represents a halogen atom, in the presence of a strong base, so as to obtain a compound with the formula IV :

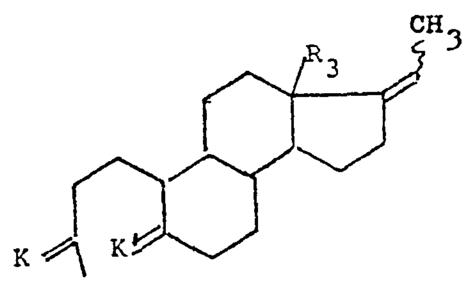

(IV)

in the form of a mixture of cis and trans isomers which is separated, if desired, into each of the isomers, then

either each of the isomers with the formula IV, or their mixture, is submitted to the action of a hydroborating agent, then to that of an oxidizing agent in alkaline medium and finally to that of a deketalizing agent and that of a cyclizing agent, so as to obtain a compound with the formula V :

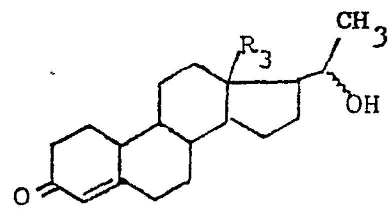

(V)

in the form of 20R or 20S isomers or their mixture,

or each of the isomers with the formula V, or their mixture, is submitted to the action of a blocking agent of the alcohol function in the form of an easily cleavable ether, so as to obtain a compound with the formula VI :

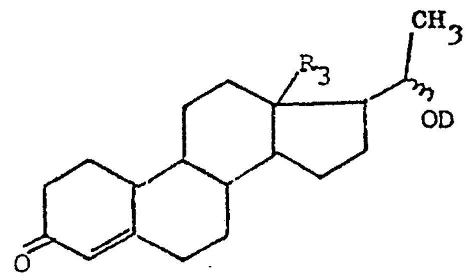

(VI)

in the form of 20R or 20S isomers or in the form of their mixture, in which OD represents an ether group,

or each of the isomers with the formula VI, or their mixture, is submitted to the action of an alkyl, alkenyl or alkynyl halide, in the presence of a base agent at low temperature, so as to obtain a compound with the formula VII :

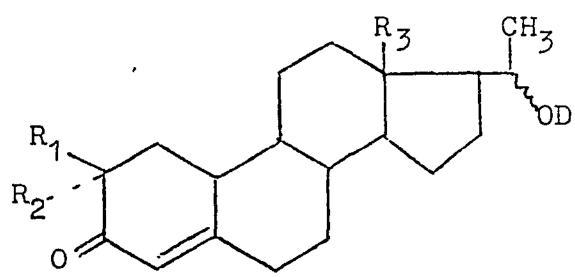

(VII)

in which $R_1$ and $R_2$ retain the same significance as in Claim 1, in the form of 20R or 20S isomers or in the form of their mixture.

**Ansprüche**

1. Die Produkte der Formel Ii :

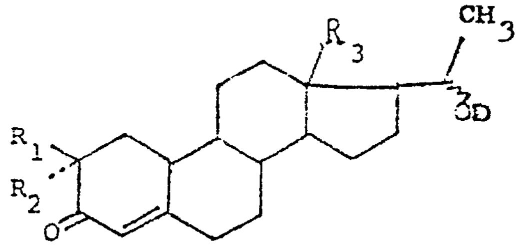

(Ii)

in Form der Isomeren 20R oder 20S oder in Form von deren Gemisch, wobei in der Formel $R_1$ und $R_2$, die gleich oder verschieden sein können, ein Wasserstoffatom, einen Alkylrest mit 1 bis 4 Kohlenstoffatomen oder einen Alkenyl- oder Alkinylrest mit 2 bis 6 Kohlenstoffatomen bedeuten, wobei lediglich einer der Substituenten $R_1$ und $R_2$ ein Wasserstoffatom bedeuten kann, oder $R_1$ und $R_2$ gemeinsam einen

Cycloalkylrest mit 3 bis 8 Kohlenstoffatomen bilden ; $R_3$ einen Alkylrest mit 2 bis 4 Kohlenstoffatomen bedeutet ; und OD eine Ethergruppe darstellt.

2. (2'R,S,20R,S)-2,2-Dimethyl-13-ethyl-20-[2'-(tetrahydropyranyl)-oxy]-18,19-dinor-pregn-4-en-3-on.

3. Verfahren zur Herstellung der Produkte gemäß Anspruch 1, dadurch gekennzeichnet, daß man durch Wittig-Reaktion eine Verbindung der Formel II :

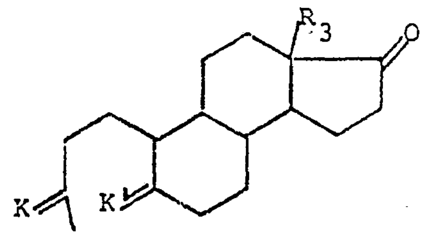

(II)

worin K und K' eine in Form des Ketals blockierte Ketongruppe darstellen und $R_3$ einen Alkylrest mit 2 bis 4 Kohlenstoffatomen bedeutet, mit einem Triphenylethylphosphonium-halogenid der Formel III :

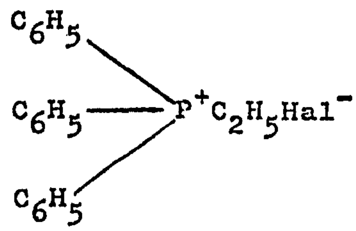

(III)

worin Hal ein Halogenatom bedeutet, in Gegenwart einer starken Base kondensiert, um eine Verbindung der Formel IV :

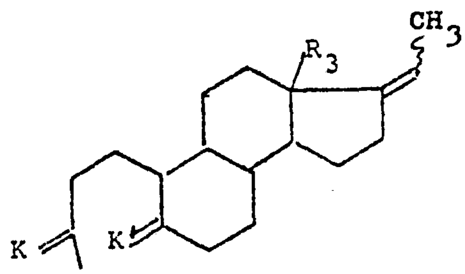

(IV)

in Form eines Gemisches der cis- und trans-Isomeren zu erhalten, das man gewünschtenfalls in ein jedes der Isomeren auftrennt, danach

entweder ein jedes der Isomeren der Formel IV oder ihr Gemisch der Einwirkung eines Hydroborierungsmittels, dann derjenigen eines Oxidationsmittels in alkalischem Milieu und schließlich derjenigen eines Diketalisierungsmittels und derjenigen eines Cyclisierungsmittels unterzieht, um eine Verbindung der Formel V :

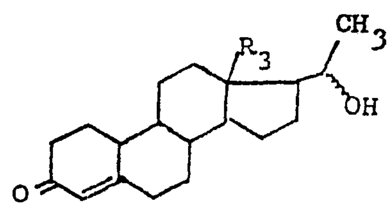

(V)

in Form der Isomeren 20R oder 20S oder in Form von deren Gemisch zu erhalten,

entweder ein jedes der Isomeren der Formel V oder deren Gemisch der Einwirkung eines Blockierungsmittels für die Alkoholfunktion in Form eines leicht spaltbaren Ethers unterzieht, um eine Verbindung der Formel VI :

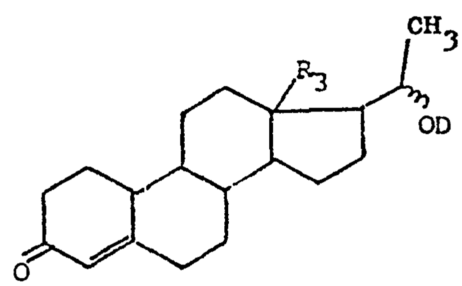

(VI)

in Form der Isomeren 20R oder 20S oder in Form von deren Gemisch zu erhalten, worin OD eine Ethergruppe bedeutet, und entweder

ein jedes der Isomeren der Formel VI oder deren Gemisch der Einwirkung eines Alkyl-, Alkenyl- oder Alkinylhalogenids in Gegenwart eines basischen Mittels bei niedriger Temperatur unterzieht, um eine Verbindung der Formel VII :

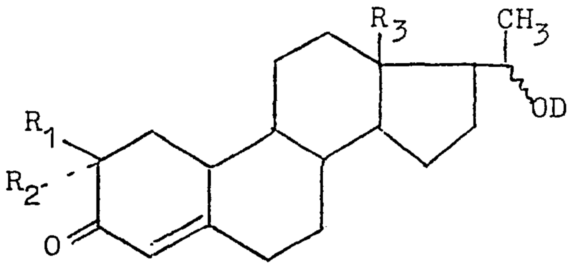

(VII)

worin $R_1$ und $R_2$ die in Anspruch 1 angegebene Bedeutung besitzen, in Form des Isomeren 20R oder 20S oder in Form von deren Gemisch zu erhalten.